# DEMANDE DE BREVET EUROPEEN

(11) **EP 2 156 854 A2**
(43) Date de publication de la demande: **24.02.2010**
(21) Numéro de dépôt: 09405140.6
(22) Date de dépôt: 18.08.2009
(51) Int. Cl.: A61M 1/00

(54) **Dispositif d'aspiration des mucosités nasales**

(30) Priorité: 20.08.2008 CH 13232008
(71) Demandeur: Dubois, Jean-Luc, 1817 Brent (CH)
(72) Inventeur: Dubois, Jean-Luc, 1817 Brent (CH)

(57) **Abrégé**

L'invention concerne un dispositif d'aspiration des mucosités nasales. Le dispositif comprend un corps (1) formant une cavité (11), une valve d'aspiration (131) reliant la cavité (11) à un embout (4) destiné à pénétrer dans la narine et une valve d'échappement (121) reliant la cavité (11) à l'extérieur; La-dite cavité (11) comprend un premier moyen destiné à évacuer l'air contenu dans la cavité (11) grâce au passage de l'air permis par l'ouverture de la valve d'échappement (121) et par la fermeture de la valve d'aspiration (131) et à aspirer les mucosités grâce au passage de l'air permis par la fermeture de la valve d'échappement (121) et par l'ouverture de la valve d'aspiration (131) en fonction de l'actionnement du premier moyen. La valve d'aspiration (131) comprend un deuxième moyen (133), différent du premier moyen, destiné à ajuster l'ouverture de la valve d'aspiration (131) en fonction de l'actionnement du deuxième moyen (133). (figure 1)

## Description

La présente invention est relative à un dispositif d'aspiration des mucosités nasales, destiné en particulier à moucher les enfants en bas âge.

Le brevet suisse n° 687 851 décrit un tel dispositif qui montre les préambules de la revendication 1. Un tel dispositif comprend un corps fabriqué dans un matériau élastique formant une cavité de compression/décompression et pourvu de deux valves. La première valve communique avec un embout amovible destiné à pénétrer dans les narines de l'enfant. La deuxième valve communique avec l'extérieur. Lorsque l'on comprime la cavité de compression/décompression, on expulse l'air de la cavité et on y génère une dépression par l'ouverture de la deuxième valve et la fermeture de la première valve. Lorsqu'on relâche la pression sur la cavité, la dépression dans celle-ci ferme la deuxième valve et ouvre la première valve en aspirant les mucosités par l'embout.

D'autres dispositifs d'aspiration des mucosités sont décrits dans EP 0 940 150 A1, EP 0 451 062 A1, DE 1 917 794, US 2,890,699 et CA 1 136 018.

L'aspiration des mucosités nasales, en particulier chez un jeune enfant, est une action qui demande une attention particulière afin qu'elle soit réalisée de manière efficace, hygiénique, mais sans blesser les narines du bébé.

L'objet de l'invention est un dispositif permettant de diminuer les risques de blesser les narines pendant la phase d'aspiration des mucosités.

L'invention atteint l'objectif par le sujet de la revendication 1. D'autres modes de réalisation sont décrits dans les revendications dépendantes.

Le principe de l'invention est un dispositif qui fonctionne en 2 étapes successives, répétables:
- premièrement, on crée dans le corps du dispositif une dépression, grâce à une action sur un premier moyen;
- deuxièmement, grâce à une action contrôlée en temps et en intensité sur un second moyen du dispositif, on libère un passage par lequel, via un embout spécifique placé dans le nez du bébé, est aspiré l'air d'une narine, chargé de mucosités.

### Figures

La figure 1 représente un premier mode de réalisation du dispositif selon l'invention avec des valves à clapets.
La figure 2 représente le dispositif de la figure 1 durant une première phase de fonctionnement.
La figure 3 représente le dispositif de la figure 1 durant une deuxième phase de fonctionnement.
La figure 4 représente un deuxième mode de réalisation du dispositif selon l'invention avec des valves à billes.
La figure 5 représente le dispositif de la figure 4 durant une première phase de fonctionnement.
La figure 6 représente le dispositif de la figure 4 durant une deuxième phase de fonctionnement.
La figure 7 représente un troisième mode de réalisation du dispositif selon l'invention avec des valves à billes, mais sans ressorts.
La figure 8 représente le dispositif de la figure 7 durant une première phase de fonctionnement.
La figure 9 représente le dispositif de la figure 7 durant une deuxième phase de fonctionnement.
Les figures 10 et 11 représentent en vue de coupe et en vue perspective, respectivement, une valve à 'lèvres' à intégrer dans une quatrième mode de réalisation du dispositif de l'invention, en position fermée, .
Les figures 12 et 13 représentent la valve à 'lèvres' des figures 10 et 11, respectivement, en position ouverte.
Les figures 14 et 15 représentent en vue de coupe latérale et en vue de coupe transversale, respectivement, un mode de réalisation d'une valve à bille, fonctionnant sans ressort, en position fermée.
Les figures 16 et 17 représentent la valve à bille des figures 14 et 15 en position ouverte.
La figure 18 représente un mode de réalisation d'une membrane élastique; servant de ressort et percée de trous permettant le passage de l'air et son principe de fonctionnement.
Les figures 19 et 20 représentent la valve à bille équipée d'un ressort à membrane élastique en vue de coupe latérale et en vue de coupe transversale en position fermée.
Les figures 21 et 22 représentent la valve à bille des figures 19 et 20 en position ouverte.

### Descriptif

Ci-après, différents modes de réalisation du dispositif selon l'invention sont décrits. Ils ont environ la même structure, tel qu'ils sont constitués d'un corps principal 1 dans lequel sont intégrées au moins deux valves agissant sous l'action de pressions exercées successivement par l'utilisateur dans les zones adéquates, mais ils sont différents essentiellement au niveau de la réalisation des deux valves. Ainsi, dans toutes les figures, les mêmes signes de référence sont utilisés pour les mêmes éléments. De même, les différentes réalisations des deux valves sont repérées avec les mêmes signes de référence.

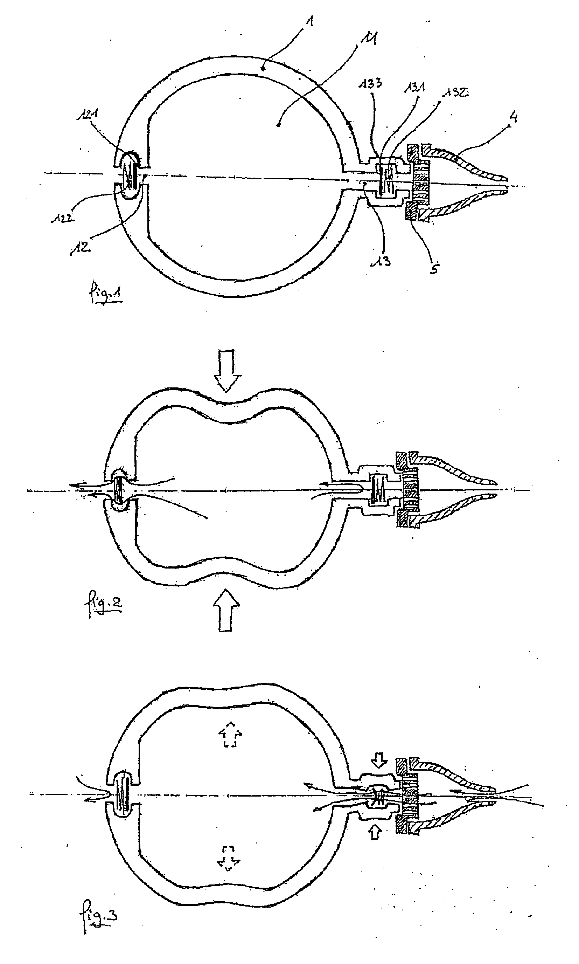

Dans les figures 1 à 22:
- les pressions exercées par l'utilisateur sont représentées par des flèches épaisses en trait plein,
- les mouvements des différents éléments tels que le corps ou les clapets sont représentés par des flèches épaisses en trait pointillé,
- les mouvements d'air sont représentés par des flèches fines en trait plein.

Dans un premier mode de réalisation du dispositif selon l'invention, les valves sont réalisées comme des valves à clapets, ci-dessous appelées simplement 'clapets', telles que représentées dans les figures 1 à 3.

Une première pression exercée sur le corps principal 1 refoule vers l'extérieur l'air contenu dans une cavité 11 via un conduit 12. Dans cette phase (figure 2), un clapet 121 s'ouvre sous l'action de la pression de l'air et libère le passage de l'air. Un deuxième clapet 131 placé dans un conduit 13 reliant la cavité 11 au nez du bébé, via un embout 4 et, optionnellement, un filtre 5, reste fermé dans cette première phase. Les clapets 121 et 131 peuvent être maintenus en place, soit par des ressorts respectivement notés 122 et 132, soit par des formes spécifiques des conduits 12 et 13.

A la fin de cette première phase, on obtient donc un dispositif dont la cavité principale 11 est en dépression.

A ce moment là, l'utilisateur insère délicatement l'embout 4 dans le nez du bébé.

La deuxième phase (figure 3) consiste en l'aspiration des mucosités. Une pression contrôlée sur une zone adéquate 133 du corps 1 permet l'ouverture plus ou moins importante du clapet 131. Cela libère un passage à travers le conduit 13, entre la cavité 11 en dépression et la narine du bébé, via l'embout 4. L'air chargé de mucosités présent dans le nez du bébé est alors aspiré vers la cavité 11 en dépression. Grâce au filtre démontable optionnel 5, les mucosités sont arrêtées avant d'entrer dans le conduit 13 et la cavité 11. A l'inverse de la première phase, le clapet 121 reste fermé, ce qui empêche l'air d'être aspiré à travers le conduit 12 vers la cavité 11 et maximise l'aspiration de l'air contenu dans la narine.

Les deux phases successives peuvent être répétées plusieurs fois de suite, jusqu'à ce que l'utilisateur juge l'opération d'aspiration des mucosités suffisante.

Il est à noter que la première phase de création de la dépression dans la cavité 11 peut être effectuée alors que l'embout 4 est dans le nez du bébé: en effet, le clapet 131 fermé empêche le refoulement de l'air vers la narine du bébé.

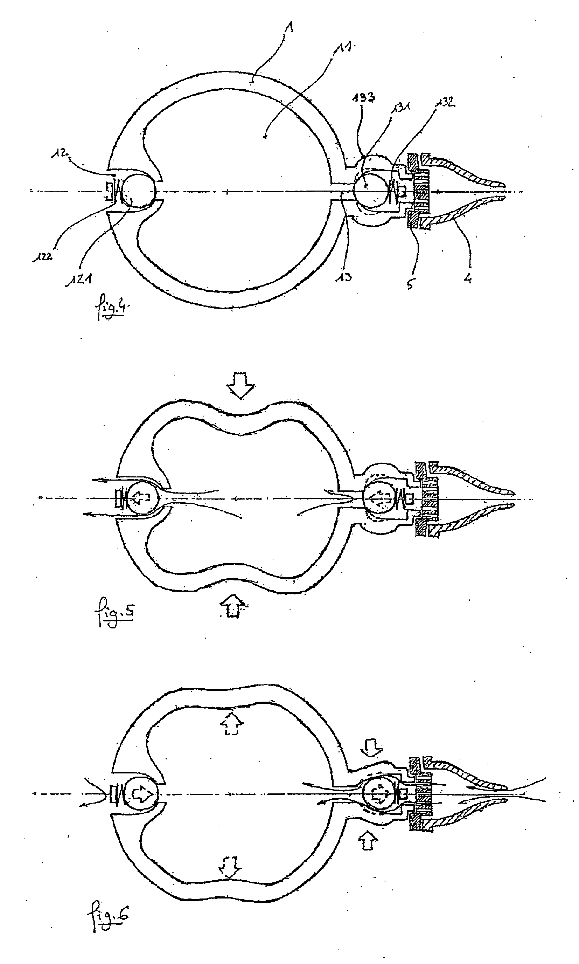

Dans un deuxième mode de réalisation du dispositif selon l'invention, les valves peuvent être des valves à billes 121 et 131, telles que représentées dans les figures 4 à 6. Celles-ci prendront place dans des logements dont les formes seront étudiées afin que, mues par les différents déplacements d'air, les billes bloquent le passage de l'air (clapet 131 dans la première phase (figure 5) de l'opération, clapet 121 dans la deuxième phase (figure 6)) ou, au contraire, permettent le passage de l'air (clapet 121 dans la première phase de l'opération, clapet 131 dans la deuxième phase).

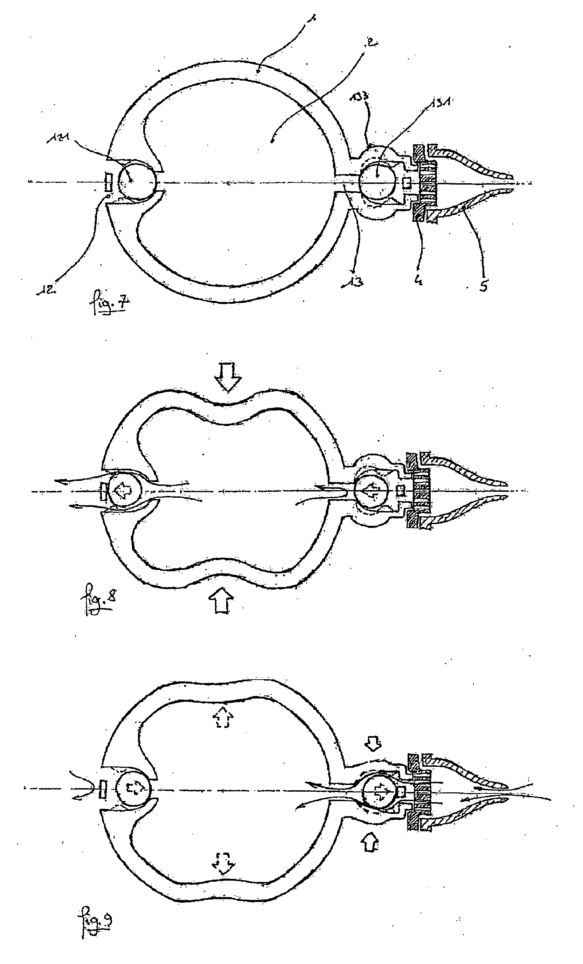

Dans le deuxième mode de réalisation du dispositif, les billes 121 et 131 sont maintenues en position fermée grâce à des ressorts notés respectivement 122 et 132 (figures 4 à 6). Un troisième mode de réalisation du dispositif selon l'invention permet de s'affranchir de ces ressorts 122 et 132 et de simplifier le dispositif, tel que représenté dans les figures 7 à 9. Une forme adéquate autour de chacune des billes 121 et 131 maintient celui-ci fermé dans la première phase (figure 8) de l'opération. Cette évolution est aussi pertinente dans le cas des valves à clapets.

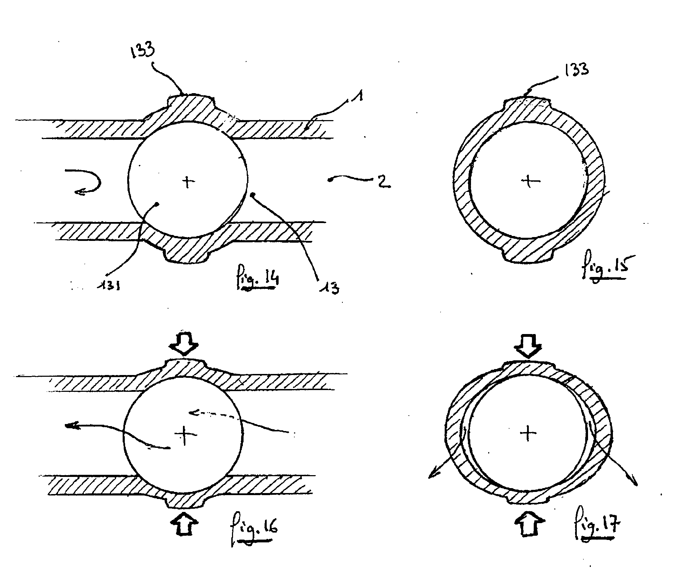

Un exemple de réalisation de la valve à bille 131 dans le conduit 13 est représenté dans les figures 14 à 17. La position au repos est représentée dans les figures 14 et 15: le contact entre la valve à bille 131 et la paroi du conduit 13 ne permet pas le passage de l'air . L'appui contrôlé sur le moyen 133 déforme la paroi du conduit 13 et crée un passage autour de la bille 131, par lequel l'air peut circuler. Le relâchement de la pression exercée sur le moyen 133 rétablit le contact entre la valve à bille 131 et la paroi du conduit 13, et bloque le passage de l'air.

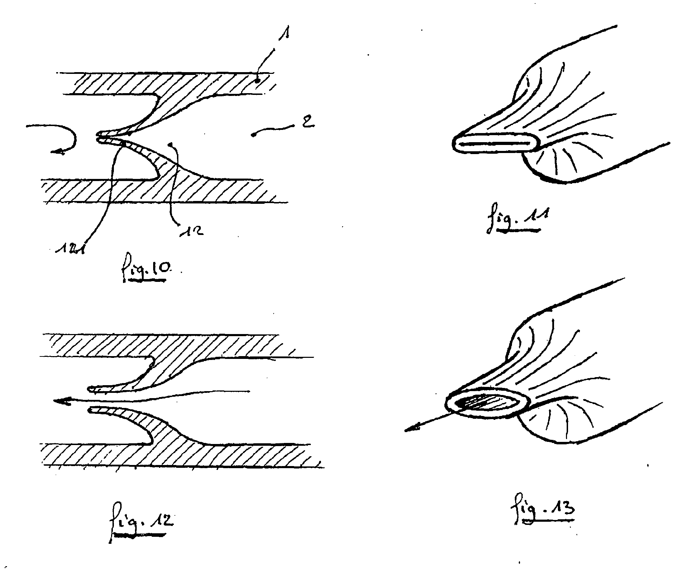

Dans un quatrième mode de réalisation du dispositif, les valves peuvent être des valves 'à lèvres', telles que représentées dans les figures 10 et 11. Cela peut être, en particulier, le cas du clapet 121. L'un au moins de ces clapets 'à lèvres' 121 et 131 peut être soit intégré au corps 1, soit rapporté dans celui-ci.

Voici le détail du fonctionnement de ce type de clapet: lors de la première phase, les lèvres du clapet 121, orientées vers l'extérieur du corps 1, s'ouvrent sous la pression de l'air expulsé par l'appui de la main de l'utilisateur sur le corps 1 . Entre les deux phases, les lèvres du clapet restent préférentiellement fermées. Lors de la deuxième phase (figures 12 et 13), les lèvres du clapet 121 restent fermées, l'air ayant tendance à entrer dans le corps 1, alors en dépression. On comprendra mieux le fonctionnement de cette solution en se reportant aux figures 10 à 13.

Il est envisageable d'utiliser ce type de clapet 'à lèvres' également pour le clapet 131. Dans ce cas, les lèvres sont orientées vers l'intérieur du corps 1. Les fonctionnements sont donc inversés par rapport au clapet 121: le clapet 131 est fermé lors de la première phase (figures 10 et 11) et ouvert lors de la deuxième phase (figures 12 et 13).

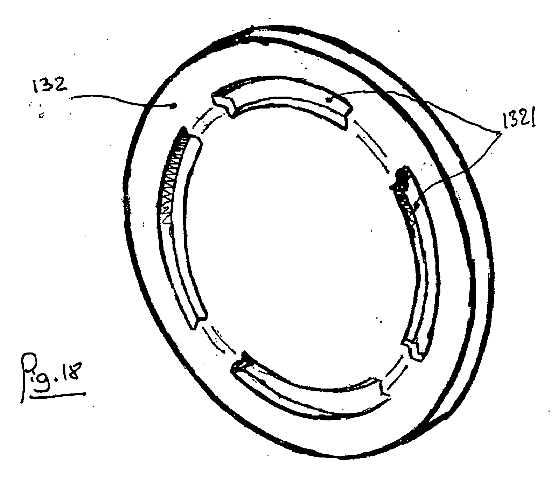

Une solution élégante pour la réalisation du (ou des) ressort(s) 122 et 132 consiste en l'utilisation d'une membrane souple qui maintient naturellement le clapet 121 ou 131 en position fermée, et dans laquelle sont percées des ouvertures placées de manière à permettre le passage de l'air durant les phases le nécessitant. Un exemple de réalisation est décrit dans les figures 18 à 22. Ce type de ressort est particulièrement favorable dans le cas du ressort 132 et quand la valve est une valve à bille.

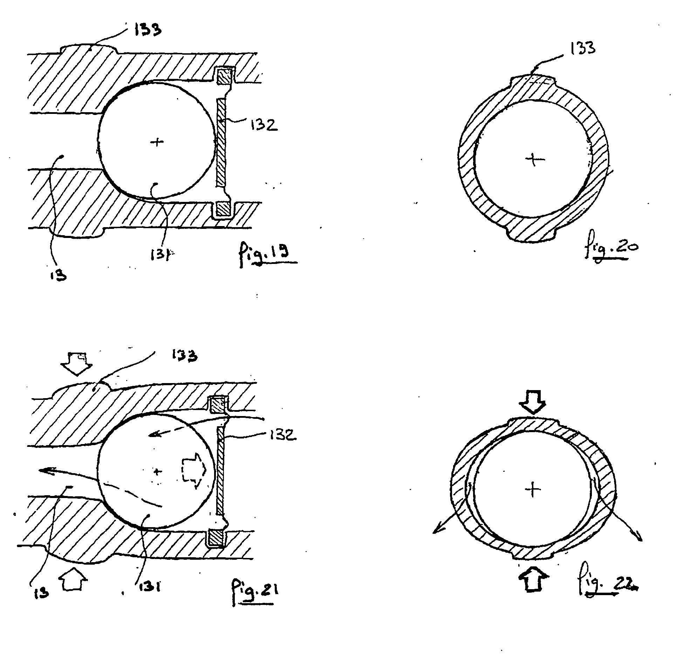

La position au repos du ressort à membrane 132 repousse la valve à bille 131 dans un logement de forme conjuguée à la bille et empêche le passage de l'air (figures 19 et 20). L'appui sur le moyen 133 déplace la valve à bille 131, en libérant le passage de l'air, contre le ressort à membrane 132 et le repousse (figures 21 et 22). Les trous 1321 ménagés dans la membrane permettent le passage de l'air à travers celle-ci. Le relâchement de la pression exercée sur le moyen 133 libère le ressort à membrane 132 qui repousse à nouveau la valve à bille 131 dans son logement, bloquant le passage de l'air.

On voit donc à la lecture de cette description un avantage important du dispositif selon l'invention: le corps 1, dans lequel sont noyés les éléments permettant le bon fonctionnement du dispositif (conduits 12 et 13, clapets 121 et 131, ressort 132), peut être monobloc.

On peut donc donner au corps 1 une forme optimale, relativement éloignée d'une forme purement fonctionnelle. Ainsi, on peut envisager une forme ludique, rassurante, à même de ne pas effrayer le bébé.

L'embout 4 et le filtre optionnel 5 restent à l'extérieur, de manière à faciliter leur montage/ démontage, que cela soit par emboîtement, clipsage ou vissage. Ainsi ces deux éléments pourront être facilement essuyés, nettoyés, lavés et/ou stérilisés.

On voit que les pressions successives sur les zones appropriées du corps 1 permettent le bon fonctionnement du dispositif. Il apparaît donc favorable de réaliser le corps principal 1 en un matériau souple, par exemple un élastomère, de manière à ce que cela soit la déformation du matériau qui entraîne la déformation de la paroi du conduit 13 autour du clapet 131, particulièrement si celui-ci est une bille, et permette le passage contrôlé de l'air.

De plus, les formes internes du corps 1, en particulier au niveau des conduits 12 et 13 et des clapets 121 et 131, rendent difficile la réalisation en une seule partie du corps principal 1, entre autres dans le cas d'un moulage plastique, mode d'obtention du dispositif semblant le plus favorable. Il peut être également pertinent de réaliser le corps principal 1 en deux parties qui seront ensuite assemblées de manière étanche, par exemple par emboîtement serré, collage ou soudage à ultrasons, afin de permettre le bon fonctionnement du dispositif.

On a vu que les dispositifs de ce type gagnaient, pour des raisons d'hygiène, à être stérilisables. Le fait que le dispositif selon l'invention soit démontable permet un nettoyage et un lavage aisés. De plus, l'utilisation de matériaux appropriés permettant la stérilisation est envisageable pour le corps principal 1, les clapets 121 et 131 et ressorts, et surtout l'embout 4 et le filtre 5 optionnel qui sont au contact du bébé et des mucosités.

De plus, dans le contexte de l'évolution des mentalités des consommateurs, il peut être favorable de réaliser un ou plusieurs éléments du dispositif en matériaux recyclables.

Un dispositif comme décrit ci-dessus réunit donc des qualités de contrôle de la quantité et de la vitesse d'aspiration des mucosités. De plus, ce dispositif peut être utilisé d'une seule main (l'autre servant à maintenir l'enfant), être facilement nettoyable et stérilisable, pour des questions d'hygiène. Il peut également être simple d'utilisation, de montage et de démontage.

Le mécanisme permettant les mouvements d'aspiration/refoulement de l'air est préférentiellement noyé dans le corps principal 1 du dispositif. Les seuls éléments extérieurs sont les éléments démontables (afin de pouvoir être nettoyés et stérilisés) tel que l'embout 4 et, optionnellement, le filtre 5 à mucosités.

## Revendications

1. Dispositif d'aspiration des mucosités nasales, comprenant un corps (1) formant une cavité (11), une valve d'aspiration (131) reliant la cavité (11) à un embout (4) destiné à pénétrer dans la narine, et une valve d'échappement (121) reliant la cavité (11) à l'extérieur; la dite cavité (11) comprend un premier moyen destiné à évacuer l'air contenu dans la cavité (11) grâce au passage de l'air permis par l'ouverture de la valve d'échappement (121) et par la fermeture de la valve d'aspiration (131) et à aspirer les mucosités grâce au passage de l'air permis par la fermeture de la valve d'échappement (121) et par l'ouverture de la valve d'aspiration (131) en fonction de l'actionnement du premier moyen, **caractérisé en ce que** la valve d'aspiration (131) comprend un deuxième moyen (133), différent du premier moyen, destiné à ajuster l'ouverture de la valve d'aspiration (131) en fonction de l'actionnement du deuxième moyen (133).

2. Dispositif selon la revendication 1 ou 2, **caractérisé en ce que** au moins l'une des valves (121; 131) est une valve du type à lèvre.

3. Dispositif selon la revendication 1, **caractérisé en ce que** au moins l'une des valves (121; 131) est une valve du type à bille, la dite bille étant placée dans un emplacement de forme appropriée permettant ou bloquant le passage de l'air selon la position de la bille.

4. Dispositif selon l'une des revendications 1 à 3, **caractérisé en ce que** au moins l'une des valves (121; 131) est équipée d'un ressort (122; 132) de maintien de la valve (121; 131) en position fermée.

5. Dispositif selon la revendication 4, **caractérisé en ce, que** au moins un des ressorts de maintien (122; 132) est une membrane élastique et/ou percée.

6. Dispositif selon l'une des revendications 1 à 5, **caractérisé en ce qu'**il comprend un embout (4) montable et démontable du corps (1) et destiné à pénétrer dans la narine de l'enfant.

7. Dispositif selon l'une des revendications 1 à 6, **caractérisé en ce que** le corps (1) est réalisé en un matériau souple et le premier moyen est réalisé par le matériau souple du corps.

8. Dispositif selon l'une des revendications 1 à 7, **caractérisé en ce que** le deuxième moyen (133) est destiné à progressivement ajuster l'ouverture de la valve d'aspiration (131) entre un état fermé et un état complètement ouvert.

9. Dispositif selon l'une des revendications 1 à 8, **caractérisé en ce que** la valve d'aspiration (131) est réalisée en un matériau souple et le deuxième moyen (133) est réalisé par le matériau souple du corps (1).

10. Dispositif selon la revendication 9, étant dépendant de la revendication 2, **caractérisé en ce que** le deuxième moyen (133) est destiné à changer la position de la lèvre en fonction de la pression exercée sur le matériau souple de la valve d'aspiration (131).

11. Dispositif selon la revendication 9, étant dépendant de la revendication 3, **caractérisé en ce que** le deuxième moyen (133) est destiné à changer la position de la bille en fonction de la pression exercée sur le matériau souple de la valve d'aspiration (131).

12. Dispositif selon l'une des revendications 6 à 11, **caractérisé en ce qu'**il comprend un filtre (5), destiné à bloquer les mucosités lors de leur aspiration, qui est placé entre l'embout (4) et le corps (1), ce filtre (5) étant montable et démontable du corps (1) et de l'embout (4).
